**Europäisches Patentamt**

**European Patent Office**

**Office européen des brevets**

(11) Veröffentlichungsnummer: **0 017 027 B1**

(12) **EUROPÄISCHE PATENTSCHRIFT**

(45) Veröffentlichungstag der Patentschrift: 13.04.83

(51) Int. Cl.³: **C 01 B 33/28**, B 01 J 29/06

(21) Anmeldenummer: **80101255.0**

(22) Anmeldetag: **12.03.80**

(54) Verfahren zur Herstellung eines borhaltigen Zeolithen vom Strukturtyp ZSM-5 und seine Verwendung als Katalysator.

(30) Priorität: **14.03.79 DE 2909929**

(43) Veröffentlichungstag der Anmeldung: **15.10.80 Patentblatt 80/21**

(45) Bekanntmachung des Hinweises auf die Patenterteilung: **13.04.83 Patentblatt 83/15**

(84) Benannte Vertragsstaaten: **AT BE DE FR GB IT NL**

(56) Entgegenhaltungen:
**EP-A-0 007 081**
**EP-A-0 007 098**
**DE-A-1 952 192**
**DE-A-2 450 708**
**DE-A-2 746 790**
**DE-A-2 817 576**
**DE-C-462 147**
**DE-C-463 719**
**US-A-4 049 573**
**US-A-4 108 881**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Laszlo, Marosi, Dr., Londoner Ring 11, D-6700 Ludwigshafen (DE)**
Erfinder: **Stabenow, Joachim, Dr., Am Feldrain 22, D-6940 Weinheim (DE)**
Erfinder: **Schwarzmann, Matthias, Dr., Carl-Bosch-Strasse 54, D-6703 Limburgerhof (DE)**

## Verfahren zur Herstellung eines borhaltigen Zeolithen vom Strukturtyp ZSM-5 und seine Verwendung als Katalysator

Es ist bekannt, Zeolithe vom Typ ZSM-5 aus Reaktionsmischungen, die $SiO_2$, $Al_2O_3$ und Alkali enthalten, in Gegenwart von Aminen unter hydrothermalen Bedingungen herzustellen. Als $SiO_2$-haltige Ausgangsstoffe dienen dabei in erster Linie Wasserglas, verschiedene Kieselsäuren und Kieselsäureole.

Wegen der niedrigen Raum-Zeit-Ausbeuten sind diese Verfahren kostspielig, da der Feststoffgehalt der Reaktionsmischungen nur etwa 10 Gew.-% beträgt. Die Kristallisation erfordert eine Zeit von etwa 5 Tagen.

Es wurde nun gefunden, daß man bei der Herstellung von borhaltigen Zeolithen vom Strukturtyp ZSM-5 aus $SiO_2$, Alkali und einem Oxid eines 3wertigen Metalls in Gegenwart von Aminen durch hydrothermale Kristallisation eine erhebliche Vereinfachung des Verfahrens und Verbesserung der Raum-Zeit-Ausbeute erzielt, wenn man als Ausgangsstoff ein feingemahlenes Borsilikatglas und ein Diamin verwendet.

Vorteilhaft arbeitet man bei der Durchführung des erfindungsgemäßen Verfahrens in Gegenwart von Hexamethylendiamin.

Aus der US-A-4 049 573 sind Zeolithe bekannt, die zwar Bor enthalten, das jedoch nicht durch gemeinsame Kristallisation der Ausgangsstoffe, sondern durch einfaches Vermischen beziehungsweise Imprägnieren der zunächst borfreien Zeolithe mit Borsäure eingebracht wurde.

Es ist daher überraschend, daß man zur Herstellung von borhaltigen ZSM-5-Zeolithen Reaktionsmischungen auch mit Feststoffgehalten von mehr als 25 Gew.-% kristallisieren kann, wenn man in Gegenwart von Diaminen arbeitet und als Ausgangsmaterial ein fein gemahlenes Borsilikatglas verwendet. Im Vergleich zu pyrogenen Kieselsäuren, Kieselsäuresolen oder Wasserglas gilt das Glas als reaktionsträge. Es war überraschend, daß mit Hilfe von nur schwach basischen Diaminen derartiges Glas überhaupt umgesetzt werden und dabei sogar die Raum-Zeit-Ausbeute beträchtlich gesteigert werden konnte.

Das erfindungsgemäße Verfahren kann durchgeführt werden, indem man fein gemahlenes Borsilikatglas mit einer wäßrigen Lösung eines organischen Diamins vermischt, wobei der Feststoffgehalt vorteilhaft größer als 25 Gew.-% betragen soll, und die Mischung dann 1 bis 10 Tage unter Rühren oder Schütteln auf Temperaturen zwischen 100 bis 200°C unter ihrem Eigendruck erhitzt bis Kristallisation eintritt. Die Kristalle werden von der Mutterlauge abgetrennt, gewaschen und getrocknet. Bevorzugt wird bei Temperaturen zwischen 140 bis 160°C und einer Reaktionszeit zwischen 2 bis 6 Tagen gearbeitet.

Als Diamine verwendet man insbesondere Hexamethylendiamin in wäßriger Lösung. Als Glas verwendet man besonders vorteilhaft fein gemahlene, alkaliarme Borsilikatgläser. Die erhaltenen Zeolithe weisen die Kristallstruktur des bekannten Aluminosilikatzeolith ZSM-5 auf.

Die erfindungsgemäß hergestellten Zeolithe enthalten in der Regel, je nach der Zusammensetzung des verwendeten Glases, $SiO_2$, $Al_2O_3$ und $B_2O_3$ in verschiedenen Verhältnissen, z. B. $SiO_2$ und $Al_2O_3$ im molaren Verhältnis 30 bis 200 zu 1 und $SiO_2$ zu $B_2O_3$ 3 bis 200 zu 1. Je nach verwendeter Glassorte können auch noch andere Komponenten im Zeolith enthalten sein. Auch größere Mengen des verwendeten Diamins können in den intrakristallinen Poren eingelagert sein.

Dieses Diamin kann beispielsweise durch Verbrennen aus den Poren entfernt werden, wobei katalytisch aktive Materialien entstehen. Je nach Zusammensetzung des verwendeten Glases kann der Zeolith auch einen wechselnden Alkaligehalt aufweisen. Die Alkaliionen können mit Hilfe bekannter Ionenaustauschtechniken gegen Protonen oder andere Kationen ausgetauscht werden.

Die erfindungsgemäß hergestellten Zeolithe können als Katalysatoren bei der Umsetzung von niederen Alkoholen, wie Methanol und/oder Dialkyläthern, wie Dimethyläther oder Diäthyläther zu aromatenhaltigen Kohlenwasserstoffen und/oder Olefinen verwendet werden. Außerdem kann man die Zeolithe bei Alkylierungs- und Isomerisierungsreaktionen anwenden.

Die Durchführung des erfindungsgemäßen Verfahrens wird anhand des nachstehenden Beispiels näher erläutert.

### Beispiel

45 g eines fein gemahlenen handelsüblichen Borosilikatglases, das etwa 80 Gew.-% $SiO_2$, 12,9 Gew.-% $B_2O_5$, 2,27 Gew.-% $Al_2O_3$ und 3,25 Gew.-% $Na_2O$ enthält, werden in 118 g 50prozentige wäßrige Hexamethylendiaminlösung eingetragen und für 5 Tage unter Eigendruck auf 150°C erhitzt, wobei die Mischung gerührt wird. Das kristalline Produkt wird filtriert, gewaschen und bei 100°C getrocknet. Laut Röntgenbeugungsanalyse besteht es aus gut kristallisiertem Material vom Strukturtyp des bekannten Aluminosilikatzeolithen ZSM-5.

Die chemische Analyse ergibt folgende Werte: 5,05% $B_2O_3$; 2,42% $Al_2O_3$; 78,7% $SiO$; 1,69% $Na_2O$; Rest Hexamethylendiamin/$H_2O$.

Verwendet man unter gleichen Reaktionsbedingungen in 118 g Hexamethylendiaminlösung 60 g des gleichen feingemahlenen Glaspulvers, so erhält man ebenfalls ein zu 100% kristallisiertes Endprodukt.

### Patentansprüche

1. Verfahren zur Herstellung eines borhaltigen Zeolithen vom Strukturtyp ZSM-5 aus SiO₂, Alkali und einem Oxid eines dreiwertigen Metalls in Gegenwart von Aminen durch hydrothermale Kristallisation, dadurch gekennzeichnet, daß man als Ausgangsstoff fein gemahlenes Borsilikatglas und ein Diamin verwendet und die Umsetzung zwischen 100 bis 200°C durchführt.

2. Verfahren nach Anspruch 1, dadurch gekennzeichnet, daß man Hexamethylendiamin verwendet.

3. Verfahren nach Anspruch 1 bis 2, dadurch gekennzeichnet, daß der Feststoffgehalt im Reaktionsgemisch größer als 25 Gew.-% ist.

4. Verwendung der nach Anspruch 1 bis 3 hergestellten Zeolithe vom Strukturtyp ZSM-5 zur Umsetzung von niederen Alkoholen und/oder Dialkyläthern zu aromatenhaltigen Kohlenwasserstoffen und/oder Olefinen.

## Claims

1. A process for the preparation of a boron-containing zeolite of the ZSM-5 structural type from SiO₂, an alkali and an oxide of a trivalent metal, by hydrothermal crystallization in the presence of an amine, wherein a diamine and, as the starting material, finely ground borosilicate glass are used, and the reaction is carried out at from 100 to 200°C.

2. A process as claimed in claim 1, wherein hexamethylenediamine is used.

3. A process as claimed in claims 1 and 2, wherein the solids content in the reaction mixture is greater than 25% by weight.

4. Use of the zeolites of the ZSM-5 structural type, prepared as claimed in any of claims 1 to 3, for the conversion of lower alcohols and/or dialkyl ethers to hydrocarbons which contain aromatics and/or to olefins.

## Revendications

1. Procédé de préparation d'une zéolithe contenant du bore, du type structural ZSM-5, par cristallisation hydro-thermique d'un mélange de SiO₂, d'un métal alcalin et d'un oxyde d'un métal trivalent en présence d'amines, caractérisé en ce que l'on emploie comme matières de départ un verre boro-silicaté finement moulu et une diamine et effectue la réaction entre 100 et 200°C.

2. Procédé suivant la revendication 1, caractérisé en ce que l'on emploie l'hexaméthylène-diamine.

3. Procédé suivant la revendication 1 ou 2, caractérisé en ce que le mélange réactionnel possède une teneur en matières solides supérieure à 25% en poids.

4. Utilisation des zéolithes du type structural ZSM-5, préparées par le procédé suivant l'une des revendications 1 à 3, pour la transformation d'éthers de dialcoyle et(ou) d'alcools inférieurs en hydrocarbures à fraction aromatique et(ou) oléfines.